# EUROPEAN PATENT APPLICATION

(11) **EP 2 949 313 A1**
(43) Date of publication of application: **02.12.2015**
(21) Application number: 15170115.8
(22) Date of filing: 01.06.2015
(51) Int. Cl.: A61K 8/26, A61Q 15/00, A61K 8/73, A61K 8/02

(54) **DEODORANT ANTIPERSPIRANT COMPOSITIONS**

(30) Priority: 30.05.2014 US 201462005010 P; 13.04.2015 US 201514684695
(71) Applicant: Johnson & Johnson Consumer Companies Inc., Skillman, NJ 08558 (US)
(72) Inventor: LEMOS ANCONI, Glasiela, 12245-500 Sao Jose dos Campos -SP (BR); DE CASTRO MONTEIRO LOFFREDO, Luciana, 12242-431 Sao Jose dos Campos - SP (BR)
(74) Representative: Carpmaels & Ransford LLP

(57) **Abstract**

The present invention relates to physically stable, silicone-free, antiperspirant compositions that are effective for 72 hours and comprise an oil absorbent selected from the group consisting of aluminum starch octenylsuccinate, tapioca starch, and polymethylsilsesquioxane; silica; an anti-perspirant; an ether compound; and at least one surface active agent having a mean HLB value from about 5 to about 12.

## Description

### Field of the Invention

The present invention relates to physically stable, silicone-free, antiperspirant compositions. The compositions are effective for antiperspirant action for 72 hours.

### Background of the Invention

The present invention relates to deodorant antiperspirant compositions that can be dispensed by roll-on. Many roll-on deodorant or antiperspirant compositions have been based on a low molecular weight alcohol such as ethanol as the principal carrier and/or active ingredient in the composition, by which herein we mean the ingredient providing the largest single weight fraction. Ethanol has bactericidal properties and is a good solvent to common antiperspirant actives. However, it has a number of undesirable characteristics including stinging, especially if the skin is broken or abraded, and a significant cooling effect. For that reason and since consumer perception can influence to a considerable extent whether or not a consumer repurchases the same brand again, it is desirable to devise compositions for consumers who wish to avoid ethanol-based compositions. Some alternative formulations are aqueous emulsions but they can suffer from one or more unfavorable characteristics such as a perception of drying too slowly and/or greasiness.

Published patent application WO03/041674 discloses a roll-on formulation of the water-in-oil type. Water-in oil formulations are known to the skilled formulator to present different problems from oil-in-water cosmetic formulations; for example, they act differently because the external phase is different. Such compositions are likely to have problems of drying discussed above.

United States Patent No. 5849276 discloses the use of silica as a nucleating agent in anhydrous antiperspirant compositions. Silica has previously been incorporated into anhydrous antiperspirant stick formulations (JP55004355) and anhydrous deodorant compositions (JP01143820). Compositions according to these inventions are likely to have drying problems also.

Some antiperspirant and deodorant compositions contain a form of silicone that aids in drying once the composition is applied to the skin. Compositions applied in a liquid form such as aerosol formulations can contain silicone, containing ingredients such as dimethicone, dimethicol and cyclomethicone. There are various side effects associated with the different silicones. For example, dimethicone may cause mild itching, burning or stinging. Other side effects may be more severe, including allergic reactions such as rash, hives, difficulty with breathing, tightness in the chest, swelling of the mouth, face, lips or tongue, severe or persistent itching, burning, stinging or worsening dryness. See, for example, the web sites www.drugs.com and www.ehow.com. Additionally, products containing silicone may have stability issues. Commercial antiperspirant roll-on products such as Unilever's REXONA Men V8, Beiersdorf's NIVEA Pearl & Beauty, GIONNAVA's GIONNAVA Baby Pink, and others require shaking prior to use. These commercial compositions separate upon standing and require mixing. They may also require the use of a ball in the container to aid in mixing the composition before use.

There is a need for a roll-on antiperspirant that is physically stable and dries quickly but does not contain silicone.

### Summary of the Invention

The present invention provides a deodorant antiperspirant roll-on composition comprising: an oil absorbent selected from the group consisting of aluminum starch octenylsuccinate, tapioca starch, and polymethylsilsesquioxane; silica; an anti-perspirant; an ether compound; and at least one surface active agent having a mean HLB value from about 5 to about 12, wherein the composition is substantially free of silicone.

Compositions according to the invention provide physical stability, fast absorbency and long lasting efficacy. The compositions dry fast and the antiperspirant and deodorant benefits may last up to 72 hours.

### Detailed Description of the Invention

The compositions of the invention are substantially free of silicone. As used herein, "substantially free of silicone" means containing less than 0.1 percent preferably less than 0.01 percent by weight silicone. In one embodiment, the compositions are completely free of silicones, i.e., contain 0% by weight silicone.

Compositions according to the invention contain an oil absorbent selected from the group consisting of aluminum starch octenylsuccinate (also known as modified corn starch), tapioca starch, and polymethylsilsesquioxane. The amount of oil absorbent may range from about 0.25 to about 0.75 percent by weight of the final formula.

Compositions according to the present invention also contain silica. The silica may be selected from silicic acids (Si(OH)₄ or SiO₂) and any type of derivatives or modifications thereof. Suitable examples include condensation products thereof, i.e., polysilicic acids ((SiO₂)ₘ×nH₂O), silicic anhydride (silica, SiO₂), fumed silica, hydrated silica (SiO₂×H₂O), silica gel and/or silicate esters. The silica may be a particulate silica, such as an amorphous silica, e.g., a fumed silica. It is particularly desirable to employ such a fumed (sometimes called pyrogenic) silica which has been hydrophobically treated. Such materials are commercially available under the name hydrophobic silica. Hydrophobic silicas are obtained by chemically bonding a hydrophbic substituent such as a siloxane group onto the surface of the silica, possibly following an intermediate treatment in which the surface of the silica has been rendered hydrophilic. Suitable reactants to generate a hydrophobic substituent include halosilanes and in particular chlorosilanes and methylated silazanes such as hexamethyldisilazane. It is particularly desirable to employ a silica that is capable of thickening an oil such as a plant oil.

Desirably, the silica, such as the fumed silica, and especially the hydrophobic silica has a BET specific surface area of at least 100 m²/g and particularly about 150 to 400 m²/g. The silica comprises very fine particles, fumed silica commonly having a diameter for individual particles of below 40 nm and in many instances at least 99% by weight of the particles have diameters below 40 nm. In fumed silica as supplied, some aggregation can occur so that in many embodiments, the supplied silica has an average particle size (diameter) of less than or equal to 1000 nm, preferably less than or equal to 500 nm, i.e., the diameter of the silica particle of average weight. In at least some desirable embodiments, at least 99% by weight of the silica particles, as supplied, have an average particle size in the range of 2 to 15 µm.

The amount of silica in the formulation is often selected taking into account the desired viscosity of the eventual formulation, together with other attributes such as its effect on the speed of drying of the formulation, its perceived greasiness and/or its perceived stickiness. The amount of silica in the composition may range from about 0.2% to about 2%, or from about 0.3% to about 1.5%, or about 0.6% to about 0.8%, or about 0.5% by weight, based on the total weight of the composition.

Compositions according to the present invention further include an ether compound. The ether compound forms an oil phase for compositions of the invention. Suitable ether compounds include, but are not limited to, C12-15 alkyl benzoate, cyclomethicone, PPG-14 butyl ether, PPG-15 stearyl ether and combinations thereof. In addition to dissolving actives and preventing recrystallization in the final formulation, the ether compound offers lubricating properties that improve the performance of roll-on balls. PPG-15 stearyl ether is stable at the very low pH levels common to antiperspirant/deodorant formulas. It is a good spreading emollient that not only moisturizes by itself but will also act as a carrier for oil soluble actives, such as beta hydroxyl acids. The amount of ether compound in the compositions of the present invention may range from about 1% to about 10% or from about 2% to about 6% by weight, based on the total weight of the composition.

Compositions according to the present invention may be oil-in-water emulsions. The oil-in-water emulsion will contain at least one solvent or vehicle. The solvent or vehicle can be selected from glycols, glycerol, polar and non-polar oils, hydrocarbons, ethers, esters, medium- and long-chain alcohols, alkoxylated alcohols, polyhydric alcohols, polyols, and mixtures thereof. Specific examples include propylene glycol, dipropylene glycol, ethylene glycol, glycerol, diglycerol, diacetin, triacetin, isopropyl palmitate, isododecane, isohexadecane, hydrogenated polydecene, triglycerides, mineral oil, and mixtures thereof. The amount of solvent or vehicle may range from about 1% to about 10% by weight, based on the total weight of the composition. The water content of the composition may range from about 50% to about 93% by weight or from about 60% to about 85% by weight, based on the total weight of the composition.

Compositions according to the present invention contain at least one surface-active agent. The surface-active agent has a mean HLB value from about 5 to about 12. These agents may assume the function of forming a coating on the exterior surface of the particles of the water-absorbing component(s), thereby influencing the surface properties thereof. More specifically, the surface-active agents aid in retaining the water absorbing agent on the skin, they stabilize the particles in the formulation and facilitate incorporation of water. Surface-active agents include compounds, including monomers, dimers, trimers, oligomers and polymers, which have lipophilic as well as hydrophilic functionalities of a strength sufficient to develop affinity to both hydrophilic and lipophilic portions of the formulation. Such agents form oriented layers around the water-absorbing particles. In this way, the particles of the water-absorbing component(s) are stabilized and distributed homogeneously within the formulation and, if necessary, protected from undesirable water absorption from a hydrous base formulation.

Emulsifiers are examples of surface active agents. Emulsifiers are often characterized by their hydrophilic/lipophilic balance or HLB. High HLB values indicate good water, or polar solvent solubility of the emulsifier while low HLB values are indicative of good solubility in non polar systems, such as oil. Compositions of the present invention include a non-ionic emulsifier or mixture of emulsifiers forming an emulsifier system. The emulsifier system typically has a mean HLB value in the region of from about 5 to about 12 or from about 6 to about 10. An especially desired mean HLB value is from about 7 to about 9. The mean HLB value can be provided by selecting an emulsifier having such an HLB value, or more preferably by employing a combination of at least two emulsifiers, a first (lower) HLB emulsifier having an HLB value in the range of from 2 to 6.5, such as from 4 to 6 and a second (higher) HLB emulsifier having an HLB value in the range of from about 6.5 to 18 and especially from about 12 to about 18. When a combination of emulsifiers is employed, the average HLB value can be obtained by a weight average of the HLB values of the constituent emulsifiers.

Suitable combinations of non-ionic surfactants include, but are not limited to, steareth-21 (high HLB) and steareth-2 (low HLB) and TWEEN 80 (high HLB) and SPAN 80 (low HLB).

Another suitable emulsifier includes a hydrophilic moiety provided by a polyalkylene oxide-8 (polyglycol), and a hydrophobic moiety provided by an aliphatic hydrocarbon, preferably containing at least 10-carbons and commonly linear. The hydrophobic and hydrophilic moieties can be linked via an ester or ether linkage, possibly via an intermediate polyol such as glycerol.

Preferably the hydrophobic aliphatic substituent contains at least 12 carbons, and is derived from lauryl, palmityl, cetyl, stearyl, olearyl and behenyl alcohol, and especially cetyl, stearyl or a mixture of cetyl and stearyl alcohols or from the corresponding carboxylic acids. It is particularly convenient to employ an emulsifier comprising a polyalkylene oxide ether.

The polyalkylene oxide is often selected from polyethylene oxide and polypropylene oxide or a copolymer of ethylene oxide and comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units a higher HLB value of above 6.5 and especially those containing at least 10 ethoxylate units. A preferred combination comprises a mixture of an ethoxylate containing 2 units and one containing from 10 to 40 units. The combination of emulsifiers may comprise steareth-2 and a selection from steareth-15 to steareth-30.

It is desirable to employ a mixture of ethoxylated alcohol emulsifiers in a weight ratio of emulsifier having a lower HLB value of <6.5 to emulsifier having a higher HLB value of >8 of from 1.5:1 to 6:1 and particularly from 2:1 to 5:1.

The total amount of emulsifiers in the composition is usually at least 1.5% and particularly at least 2% by weight. Commonly the emulsifiers are not present at above 6%, often not more than 5% by weight and in many preferred embodiments up to 4% by weight. An especially desirable concentration range for the emulsifiers is from 2.5 to 4% by weight.

Fragrance may be included in the final product. The amount of fragrance may be from about 0.1% to about 3% by weight, or from about 0.3% to about 2% by weight, based on the total weight of the composition. Examples of fragrances include:
The instant invention utilizes a concentration of an antiperspirant or deodorant active effective to reduce or control sweating or reduce or eliminate body malodour. In many desirable embodiments, the composition contains at least 1% antiperspirant active, and preferably at least 5% and often is at least 10%. Commonly, the concentration of the antiperspirant active is not higher than 30%, and in many practical embodiments is not higher than 25.5% by weight based on the total weight of the composition. A preferred concentration range for the antiperspirant active is from 10% to 20%. A most preferred concentration is 17.5%.

The antiperspirant active is conveniently an astringent aluminium and/or zirconium salt, including astringent inorganic salts, astringent salts with organic anions and complexes of such salts. Preferred astringent salts include aluminium, zirconium and aluminium/zirconium halides and halohydrate salts, such as especially chlorohydrates. Activated chlorohydrates can be incorporated, if desired. Some literature employs alternative terminology for chlorohydrates, such as basic aluminium chloride, and aluminium chlorhydrex.

Aluminium halohydrates are usually defined by the general formula Al₂(OH)ₓQ_{y}. wH₂O in which Q represents respectively chlorine, bromine or iodine, (and especially chlorine to form a chlorohydrate) x is variable from 2 to 5 and x + y = 6 while wH₂O represents a variable amount of hydration.

Zirconium actives can usually be represented by the empirical general formula: ZrO(OH)_{2n-nz}B_{z}· wH₂O in which z is a variable in the range of from 0. 9 to 2.0 so that the value 2n-nz is zero or positive, n is the valency of B and B is selected from the group consisting of chlorine (to form a chlorohydrate), other halide, sulphamate, sulphate and mixtures thereof. Possible hydration to a variable extent is represented by wH₂O. Preferably, B represents chlorine and the variable z lies in the range from 1.5 to 1.87. In practice, such zirconium salts are usually not employed by themselves, but as a component of a combined aluminium and zirconium-based antiperspirant.

The above aluminium and zirconium salts may have co-ordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl--phenylalanine, dl-valine, dl-methionine and 3-alanine, and preferably glycine which has the formula CH₂(NH₂)COOH.

In some compositions, it is highly desirable to employ complexes of a combination of aluminium chlorohydrates and zirconium chlorohydrates together with amino acids such as glycine, which are disclosed in US 3792063 (Luedders et al). Certain of those Al/Zr complexes are commonly called ZAG in the literature. ZAG actives generally contain aluminium, zirconium and chloride with an Al/Zr ratio in a i range from 2 to 10, especially 2 to 6, an Al/Cl ratio from 2. 1 to 0.9 and a variable amount of glycine. Actives of this preferred type are available from Westwood, from Summit and from Reheis.

The invention compositions can comprise, if desired, a deodorant active other than an antiperspirant active described hereinbefore. Such an alternative deodorant active can be selected conveniently from any deodorant active known in the cosmetic art such as antimicrobial actives such as polyhexamethylene biguanides, e.g., those available under the trade name COSMOCIL, or chlorinated aromatics, e.g., triclosan available under the trade name IRGASAN, non-microbiocidal deodorant actives such as triethylcitrate, bactericides and bacteriostats. Yet other deodorant actives can include bactericidal zinc salts such as zinc ricinoleate. The concentration of such alternative deodorant active is desirably from 0. 01 to 5% and in many instances is from 0. 1 to 1% by weight of the composition. In many highly desirable invention compositions, an antiperspirant active is present, either without or supplemented by the alternative deodorant active.

Compositions according to the present invention may include polyglyceryl-3 caprylate, triclosan, farnesol, trichlorocarban or combinations thereof which may function as an emollient and a deodorant by killing bacteria. TEGO® Cosmo P 813 from Evonik is a mild vegetable based cosmetic coemulsifier that may also be useful in this context. TEGO® Cosmo P 813 has anti-microbial properties. It reliably reduces odor-causing bacteria on the skin at very low concentrations. TEGO® Cosmo P 813 is an ester, with lipophylic character, which means it is an emollient to skin, an important benefit to sensitive underarm skin. It is also advantageous for body care, because while other anti-microbials have constant activity on the skin's surface, this active is activated by the surface flora associated with the sweating process. TEGO® Cosmo P 813 is cleaved by bacterial lipases, releasing caprylic acid, which functions as the antimicrobial agent.

It is preferred that the emulsions of the present invention have a viscosity which falls within a preferred range of from 1000 to 7000 mPa.s and particularly within 2500 to 5500 mPa.s. Control of viscosity is important to avoid leakage and dripping around the roller-ball or excessive running down the body during deodorant application, and for correct dose delivery. Viscosities herein are measured in a Brookfield RVT viscometer equipped with a stirrer TA and Hellipath, rotating at 20 rpm at 25°C unless otherwise stated.

Emulsions according to the present invention demonstrate a particularly desirable combination of product attributes such as improved speed of drying, superior greasiness and avoidance of excessive stickiness on application. Preferably, the emulsion is made by first preparing separate aqueous and oil mixtures which are brought together before shearing. The temperature of the respective phases can be raised, where necessary, to accelerate dissolution of the emulsifier, for example to above 50°C.

In some preferred embodiments of the present invention, the formulations are dispensed from a roll-on dispenser for a liquid including a bottle and a removable cap, the bottle having an interior and a first end which defines a housing for a rotatable spherical ball, said housing having a chamber within which the ball can rotate having an inward end in fluid communication with the interior of the bottle and including a lateral sealing ring dimensioned to prevent the ball from being urged into the interior of the bottle when subjected to inward axial force, an opposed outward end dimensioned to retain the ball and a side-wall having an interior surface extending between the outward end and the inward end of the ball having a segment projecting outside the housing, the cap having means to urge the ball axially towards the sealing ring, in which at least one fluid flow perturbator is located on the interior surface of the chamber in or outward of the sealing ring.

Herein the term "inward" when employed axially, for example in inward ends in respect of a housing intended for mounting on or an integral mounting with a bottle reservoir, refer respectively to the end adjacent to the interior of the bottle, and outward is that remote from the interior. Axial relates to an axis extending centrally through the inward and outward ends of the housing. Herein, the terms "upward," "downward," "above" and "below" when employed in respect of the dispenser and its constituent parts refer to when the dispenser is in an upright orientation, which is to say the cap is above the bottle.

### Examples

### Example 1

Using the ingredients in Table 1 below and the procedure below, a roll-on composition according to the invention was prepared.

**Table 1**

| INGREDIENT | FUNCTION | % w/w |
|---|---|---|
| A: Aqua | Solvent | 54.3 |
| B: Steareth-2 | Surfactant | 3.00 |
| C: Steareth-21 | Surfactant | 1.00 |
| D: PPG-15 Stearyl Ether | Emollient | 4.00 |
| E: Aluminum Chlorohydrate (50%) | Antiperspirant Agent | 35.0 |
| F: Aluminum Starch Octenylsuccinate | Absorbent | 0.50 |
| G: Silica | Absorbent | 0.30 |
| H: Ethylhexyglycerin; Caprylyl | Skin Conditioning | 1.0 |
| Glycol | Agent | |
| I: Polyglyceryl-3 Caprylate | Emulsifying Agent | 0.30 |
| J: Tocopheryl Acetate | Skin Conditioning Agent | 0.10 |
| K: Sodium Benzoate | Preservative | 0.50 |

### Procedure

1. Oil Phase - weighed D, B, C, I, H and J into a vessel and started heating to 75-80°C.
2. Water Phase - weighed A, E, and K into a separate vessel and started heating to 75 to 80°C.
3. Emulsification Step - added the oil phase into the water phase (both 75-80°C) and mixed for 5 minutes, then passed the composition through a homogenizer for 3 minutes.
4. Cooling Step - let the emulsion cool down until it reached 40°C. Added G and F and passed the composition through the homogenizer for 2 minutes.

### Example 2

The absorption rate of the composition of Example 1 as a roll on deodorant was determined along with three commercial products. The commercial products contained the following ingredients:

| NATURA ERVA DOCE Roll on | REXONA women nutritive Roll on | DOVE Original Roll on |
|---|---|---|
| Aqua, Aluminum Chlorohydrate, Steareth-2, Cyclopentasiloxane, Steareth-21,Canola Oil, Hydroxypropyl Starch Phosphate, Parfum, PPG-15 Stearyl Ether, DMDM Hydantoin, BHT, Bisabolol, Pimpinella Anisum Fruit Extract, Coumarin, Eugenol, hexyl Cinnamal, Limonene, Linalool | Aqua, Aluminum Chlorohydrate, Steareth-2, Helianthus Annuus Seed Oil, Parfum, Steareth-20, Silica Dimethyl Silyilate, Disodium EDTA, Caprilyc/Capric Trygliceride, Hydrated Silica, Gelatin Crosspolymer, Cellulose Gum, Sodium Benzoate, Alpha-Isomethyl Ionnone, Cynnamyl Alcohol, Citronellol, Geraniol, Limonene, Linalool | Aqua, aluminum chlorohydrate, helianthus annuus seed oil, glycerin, steareth-2, fragrance, steareth-20, disodium EDTA, alpha-isomethyl, ionone, benzyl benzoate, benzyl salicylate, butylphenyl methylpropional, citronellol, geraniol, hexyl cinnamal, hydroxycitronellal, linalool |

Absorption by skin is defined as the retention property of oily materials by skin without its transference when in contact with a substrate with high affinity for oily materials. The product absorption rate was determined by a combination of two techniques: in vitro determination of the solid amount in the product under specific conditions of humidity and temperature, simulating a real condition of product application on skin and determination of the amount of exogenous free oily material on the skin, after the product application and drying. The quantity of product "absorbed" by skin is calculated by the difference between the amount of solids initially applied on skin and the amount of free exogenous oily material on skin. The results are shown in Table 2 below.

**Table 2**

| | Example 1 | DOVE Original | NATURA ERVA DOCE | REXONA Women |
|---|---|---|---|---|
| Product application area | 5 cm² | 5 cm² | 5 cm² | 5 cm² |
| Quantity of product applied (Mp) | 20 mg | 20 mg | 20 mg | 20 mg |
| Amount of solids in the product (S%) | 36.67% | 40.38% | 43.03% | 40.81% |
| Quantity of free oily material after product drying (Mo%) | 1.5% | 3.1% | 3.3% | 3.3% |
| Quantity of product absorbed by skin Mp (S% - Mo%) | 2.192 | 0.938 | 1.003 | 0.781 |
| Product drying time -t (minutes) | 3 | 3 | 3 | 3 |
| Absorption Rate (mg/minute) | 0.73 | 0.31 | 0.33 | 0.26 |

Example 1 according to the invention showed an absorption rate of 0.73 mg/minute, which was 2.8 times higher than the REXONA Woman product, 2.3 times higher than the DOVE Original product and 2.2 times higher than the NATURA ERVA DOCE product.

### Example 3

Studies were performed to determine 24, 48 and 72 hour deodorant efficacy (sniff test) and 72 hour antiperspirant efficacy on a composition of the invention.

After a 20-day wash-out period, 150cm² areas - randomly distributed - were demarcated on the subjects' underarms. A composition was prepared using the ingredients in Table 3 and the procedure below. The product was applied to one underarm and the other was kept as the control (no product application). Applications were performed on 3 consecutive days. After three days of application, 24 and 48 hours after the third product application, subjects went through the axillary odor evaluation and 72 hours after the third product application, subjects went through the axillary odor evaluation and a dry hot room for 80 minutes for collecting sweat.

The wash-out period lasted 20 days. During this period the subjects were instructed to use only the standard soap and the standard bacteriostatic deodorant provided by ALLERGISA, and not to use any other type of product on the underarms. Seven days before the start of test product application (14th day of wash-out period), the subjects were instructed to stop the use of the standard deodorant and not to use any other product on the axillary region.

**Table 3**

| **INGREDIENT** | **FUNCTION** | **% w/w** |
|---|---|---|
| A: Aqua | Solvent | 53.29 |
| B: Steareth-2 | Surfactant | 3.00 |
| C: Steareth-21 | Surfactant | 1.00 |
| D: PPG-15 Stearyl Ether | Emollient | 4.00 |
| E:Aluminum Chlorohydrate | Antiperspirant Agent | 35.0 |
| F:Aluminum Starch Octenylsuccinate | Absorbent | 0.50 |
| G: Silica | Absorbent | 0.30 |
| H: Ethylhexyglycerin; Caprylyl Glycol | Skin Conditioning Agent | 1.0 |
| I: Polyglyceryl-3 Caprylate | Emulsifying Agent | 0.30 |
| J: Tocopheryl Acetate | Skin Conditioning Agent | 0.10 |
| K: Sodium Benzoate | Preservative | 0.50 |
| L: Gossypium Herbaceum Seed Oil | Skin Conditioning Agent | 0.01 |

### Evaluation of Aluminum Residues

Previous to the supervised axillary wash, the subjects' underarms were cleaned with a cotton swab, which was used for an aluminum analysis, in order to determine if there was non-authorized use of antiperspirant products or presence of residues.

### Supervised axillary washing

Subjects were instructed and supervised by a trained technician to wash their underarms, before each application of the test product, according to the following procedure: a - Wash one of the underarms for 10 seconds with an aqueous solution of standard soap 2.0%; b - Rinse thoroughly with running water until the soap is completely removed; c - Dry the underarm with a paper towel; d - Repeat the procedure with the other underarm.

### Product application

The test product was applied as previously described, in a marked area of approximately 150 cm². This procedure was repeated during three consecutive days.

### Distribution of T-shirts

The T-shirts (100% cotton) used in the test were initially washed with water and neutral soap, without fragrance. Previous to their delivery to the subjects, the T-shirts were again washed only with water. On the third day of test, right after the third product application, the subjects were given a white T-shirt provided by the researcher. Subjects were instructed not to remove the T-shirt until the odor assessment was performed.

### Axillary Odor Evaluation

Three trained technicians performed evaluations of axillary odor 24, 48 and 72 hours after the third product application, in order to evaluate the intensity of axillary odor. The voluntary subjects were instructed to enter, one at a time, into the assessment room, followed by one of the trained technicians. In order to minimize interferences of external odors, the voluntary subjects were instructed to take off the T-shirt during the assessment. During the procedure the technician used, in order to standardize the distance of each assessment, a virgin disposable glass without its bottom. For odor assessment, an 11-point scale was used, according to Table 3 (Standard Practice for the Sensory Evaluation of Axillary Deodorancy. ASTM Standards, E 1207-87 (re-approved 1993).

### Sweat Collection

72 hours after the third product application, subjects went through an exposure to heat (dry hot room). There was an 80-minute period of exposure to heat. Pads stored in closed polyethylene bags were used for collecting sweat. During the hot room session, the pads were removed from their bags and placed immediately onto the subjects' underarms after the first 40 minutes, being replaced every 20 minutes. Standardized pads were used on the underarms, as well as appropriate clothing during the Hot Room sitting. The temperature was 37.8°C ± 1°C and relative humidity was between 30% and 40%. Subjects ingested 200 mL of water when entering the Hot Room and also 40 minutes later, in order to maintain the internal level of hydration, allowing the sweat to flow freely and evidencing only the effects of the formulation of the product tested. The initial period corresponding to 40 minutes of exposure to heat was considered as being acclimatization period. The composition presented 24, 48 and 72-hour deodorant activity.

The composition provided 72-hour antiperspirant activity.

A non-exhaustive list of aspects of the disclosure is set out in the following numbered clauses:
Clause 1. A deodorant antiperspirant composition comprising:
   an oil absorbent selected from the group consisting of aluminum starch octenylsuccinate, tapioca starch, and polymethylsilsesquioxane;
   silica;
   an antiperspirant;
   an ether compound; and
   at least one surface active agent having a mean HLB value from about 5 to about 12, wherein the composition is substantially free of silicone.
Clause 2. The composition of clause 1 containing about 0.25 to about 0.75 % by weight of oil absorbent.
Clause 3. The composition of clause 1 containing about 0.2 to 2 % by weight of silica.
Clause 4. The composition of clause 1, wherein the ether compound is selected from the group consisting of C12-15 alkyl benzoate, cyclomethicone, PPG-14 butyl ether, PPG-15 stearyl ether and combinations thereof.
Clause 5. The composition of clause 1 containing about 1 to about 10% by weight of ether compound.
Clause 6. The composition of clause 1 in the form of an oil-in-water emulsion.
Clause 7. The composition of clause 1, wherein the antiperspirant is selected from the group consisting of astringent aluminum and/or zirconium salts.

## Claims

1. A deodorant antiperspirant composition comprising:
an oil absorbent selected from the group consisting of aluminum starch octenylsuccinate, tapioca starch, and polymethylsilsesquioxane;
silica;
an antiperspirant;
an ether compound; and
at least one surface active agent having a mean HLB value from about 5 to about 12, wherein the composition is substantially free of silicone.

2. The composition of claim 1 containing about 0.25 to about 0.75 % by weight of oil absorbent.

3. The composition of claim 1 or claim 2 containing about 0.2 to 2 % by weight of silica.

4. The composition of any of the preceding claims, wherein the ether compound is selected from the group consisting of C12-15 alkyl benzoate, cyclomethicone, PPG-14 butyl ether, PPG-15 stearyl ether and combinations thereof.

5. The composition of any of the preceding claims containing about 1 to about 10% by weight of ether compound.

6. The composition of any of the preceding claims in the form of an oil-in-water emulsion.

7. The composition of any of the preceding claims, wherein the antiperspirant is selected from the group consisting of astringent aluminum and/or zirconium salts.
